# EUROPÄISCHE PATENTSCHRIFT

(11) **EP 1 707 163 B1**
(45) Veröffentlichungstag und Bekanntmachung des Hinweises auf die Patenterteilung: **25.11.2009**
(21) Anmeldenummer: 05006717.2
(22) Anmeldetag: 29.03.2005
(51) Int. Cl.: A61F 5/08

(54) **Vaginaler Stützkörper**
Vaginal Support
Support vaginal

(43) Veröffentlichungstag der Anmeldung: 04.10.2006
(73) Patentinhaber: Fuchs, Karl, 8740 Zeltweg (AT)
(72) Erfinder: Fuchs, Karl, 8740 Zeltweg (AT)
(74) Vertreter: Gibler, Ferdinand

(56) Entgegenhaltungen:
- FR-A- 2 082 679
- GB-A- 226 507
- US-A- 2 365 296
- US-A- 5 687 429
- US-A1- 2003 120 178

## Beschreibung

### Gegenstand der Erfindung:

In die Scheide einzuführender Formkörper mit Stützfunktion für die Gebärmutter und davon abgeleitete Formänderungen des Stützkörpers für integrierte Tamponfunktion oder mit Urinaufnahmefunktion bei Inkontinenz.

### Gebiet der Erfindung:

Die Erfindung betrifft einen Gebärmutterhalter in Art eines mit einem Tampon vergleichbaren in den vorderen Teil der Scheide einzuführenden, zum längeren Verbleib in der Scheide bestimmten und entsprechend anatomisch angepasst geformten Stützkörpers, der die Fähigkeit hat, die Gebärmutter so wirkungsvoll zu stützen, dass es selbst bei erheblicher Gebärmuttersenkung nicht zu einem Absenken oder Heraustreten der Gebärmutter kommt. Der Stützkörper selbst wird durch einen normalen Slip in Position gehalten.
Durch unterschiedliche Bauart kann die Funktion des Stützkörpers erheblich erweitert werden. Der Innenraum von besonderen Bauarten des Stützkörpers soll zur Aufnahme von Körperflüssigkeiten, besonders für Urinabsorbierung, genutzt werden.

### Hintergrund der Erfindung:

Der Erfindung liegt die Aufgabe zugrunde, die besondere Form eines aus körperfreundlichen Kunststoffen hergestellten Stützkörpers aufzuzeigen, der überraschenderweise nach einfachem Einführen in die Scheide die gesenkte Gebärmutter (Uterus-prolaps) zurück schiebt, wobei ein normaler Slip zum Positionieren und Halten des erfindungsgemäßen Stützkörpers genügt. Dieser Stabilisierungseffekt wird durch die erfindungsgemäße anatomisch angepasste Formgebung bewirkt, die im Anspruch 1 beschrieben wird. Die bisher für diesen Zweck bekannt gewordenen Stützkörper weisen erhebliche Unzulänglichkeiten auf, insbesondere was den Komfort bei der Anwendung betrifft. So beschreiben die DE PS 38 00 744, die DE-OS 42 25 520, das EP 0663 197 B1 sowie das Deutsche Gebrauchsmuster G 92 09 403.1 sowie die US PS 4 307 716 jeweils aufblasbare bzw. flüssig befüllbare Formkörper die nach Einführen in die Scheide anschließend expandiert werden müssen. Diese Methode soll ein besonders schmerzfreies Einführen ermöglichen, ist aber sehr wenig praktikabel, insbesondere wenn beim Urinieren zunächst der Füllkörper zum Entnehmen durch Entleerung verkleinert werden muss und anschließend nach Wiedereinführung wieder befüllt werden muss. Die US PS 1 750 188 beschreibt eine Vorrichtung, die nach Einführen in die Scheide durch eine Drehbewegung an einem außen liegenden abnehmbaren "DrehKnopf" "Flügel" ausbreitet, die eine Stabilisierung in der Scheide bewirken sollen.
Alle bisher bekannten Vorrichtungen sind gekennzeichnet durch den Nachteil, dass ihre Form zwecks Stabilisierung in der Scheide nach der Einführung verändert werden muss. Dies bedingt immer eine relativ komplizierte Gestaltung, die nicht als kostengünstige und hygienische "Einweg"-Ausführung realisiert werden kann. Zudem ist eine schmerzfreie Handhabbarkeit von Ausführungsformen mit beweglichen Teilen (z. B. ausdrehbaren Flügeln) nicht gesichert.

Ein weiteres Beispiel von einem Formkörper des Stands der Technik ist in US-5 678 429 beschrieben.

Die erfindungsgemäße Stützvorrichtung kann aufgrund ihrer besonderen Form ohne vorherige "Verkleinerung" schmerzfrei eingeführt werden und dennoch genügend Halt bieten. Sie nutzt zu ihrer Stabilisierung zeitgemäße eng anliegende Unterwäsche (Slips) und kann aufgrund ihrer Formgebung auf weitere Stabilisierungshilfen verzichten. Die Gestaltung ohne bewegliche Teile erlaubt eine Massenproduktion zu minimalen Kosten, die eine Verwendung als "Einweg"- Produkt wirtschaftlich sinnvoll macht.

### Beschreibung:

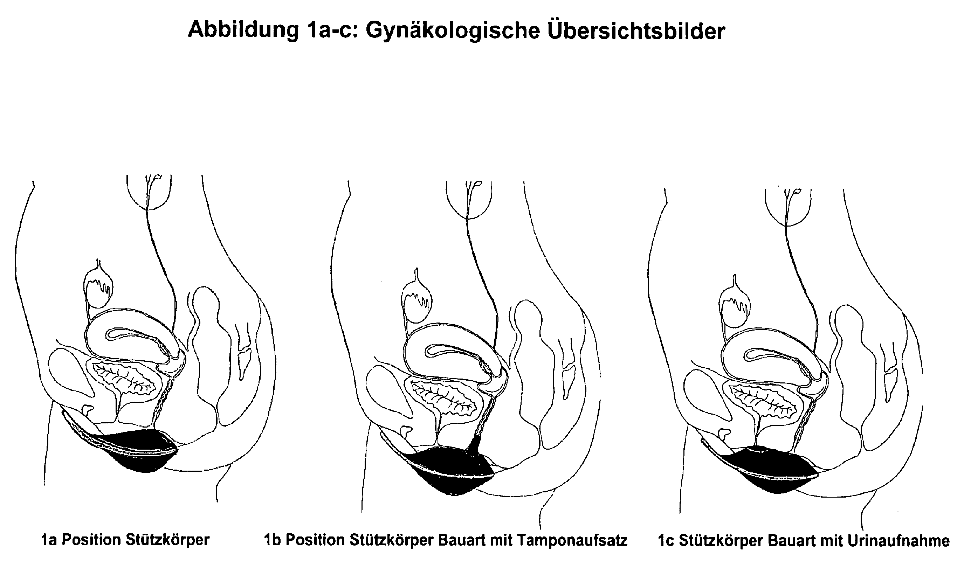

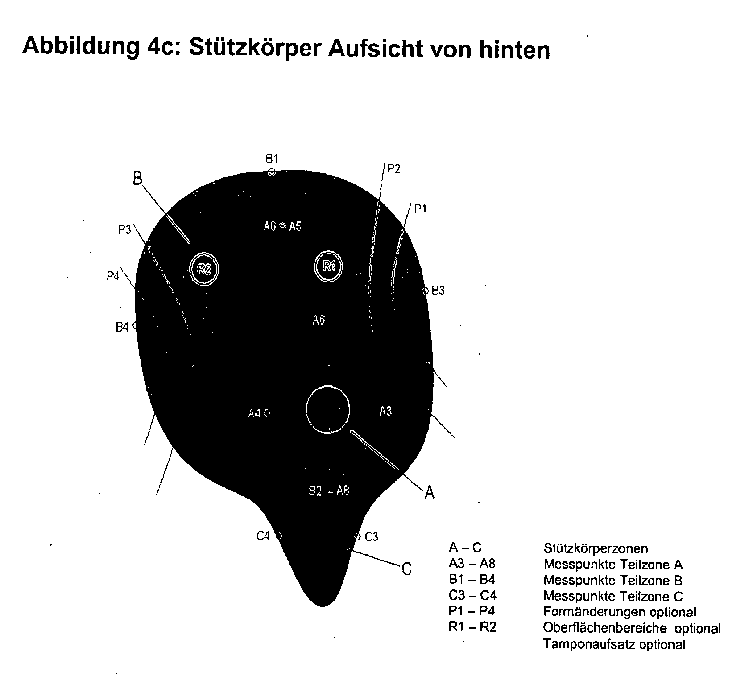

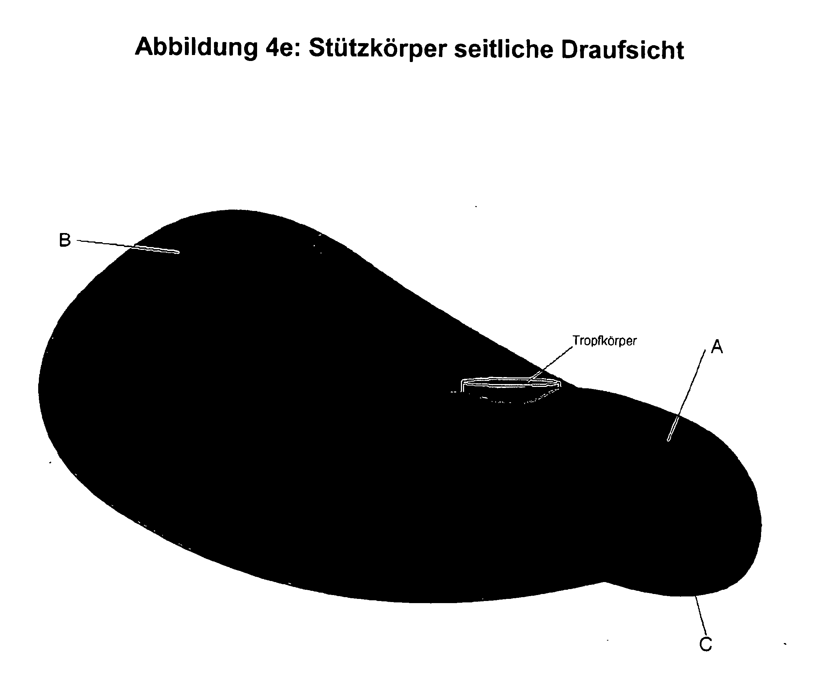

Die erfindungsgemäße Stützvorrichtung ist in den Abbildungen 1 bis 4e gezeigt und erläutert, wobei Abb. 1a-c übersichtsartig die Positionierung der erfindungsgemäßen Stützvorrichtung in unterschiedlicher Bauartvariante im Körper zeigt. Zur Erläuterung der Wirkungsweise des Stützkörpers werden drei wesentliche funktionelle Zonen des Stützkörpers gedanklich separiert und getrennt von innen (= körperseitig) nach außen erläutert.

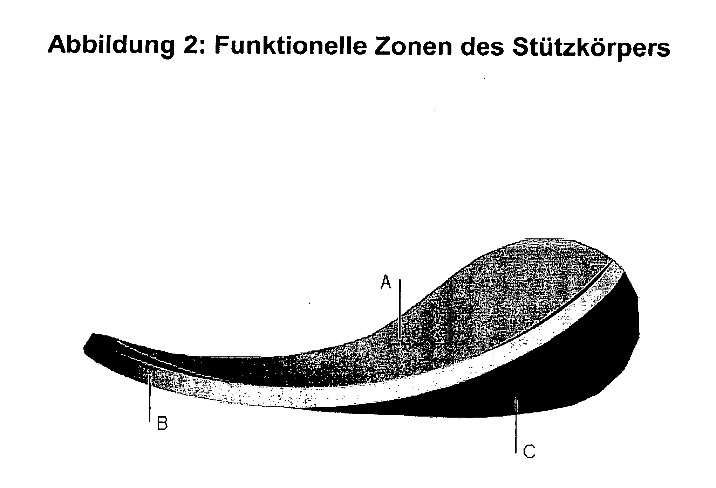

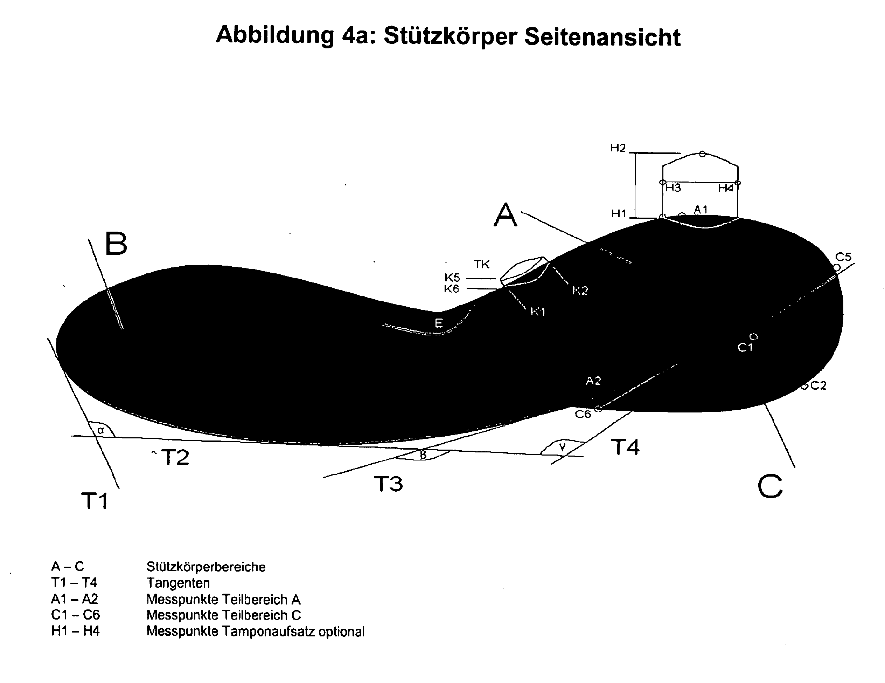

Die Zone A (Abb. 2, 3a, 4a) in der Form eines gebogenen langgestreckten Tropfens hat zwei wesentliche Funktionen und zwar i) das Zurückdrängen der Gebärmutter mittels des Tropfen-Endes (= dicker Teil des Tropfens) und ii) der Vertikalstabilisierung und Positionierung durch den dünn auslaufenden und etwas abgeflachten Teil des gebogenen Tropfens der am Beginn der Schamlippen positioniert ist. Das Tropfenende kann als Bauartvariante in Form eines Tampons ausgeformt sein. Die körpemahe Seite des Tropfens kann ebenfalls als Bauartvariante mit einer Öffnung als Tropfkörper zur Aufnahme des Urins versehen sein.

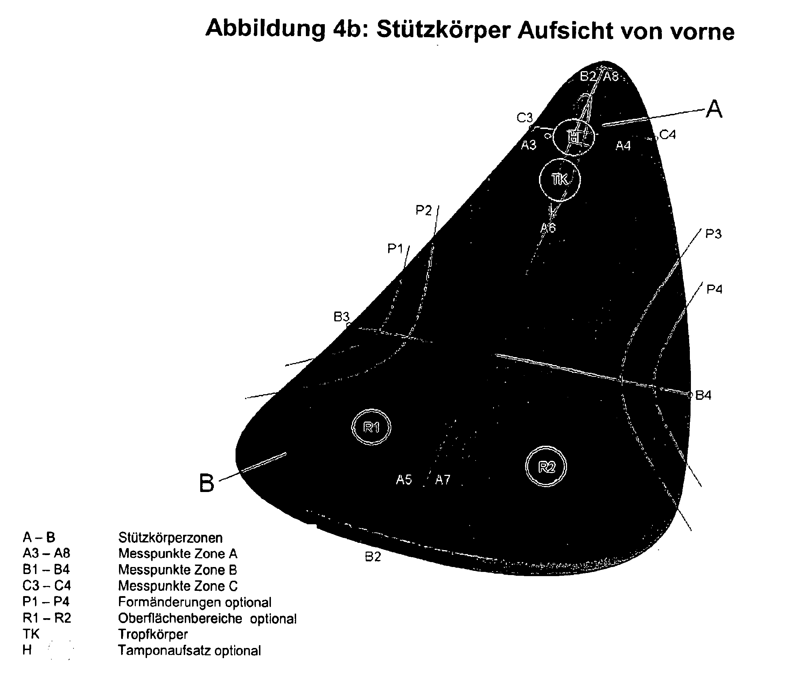

Die **Zone B** (Abb. 2, 3a, 3b, 4a, 4b) in der Form eines flachen, gebogenen langgestreckten Herzens. Der obere (= breite) Teil des Herzens ist auf den Nabel, der untere (= schmale) Teil auf den Anus gerichtet. Er hat die Funktion der horizontalen Stabilisierung und Positionierung, bewirkt aber auch noch eine weitere vertikale Stabilisierung und Positionierung.

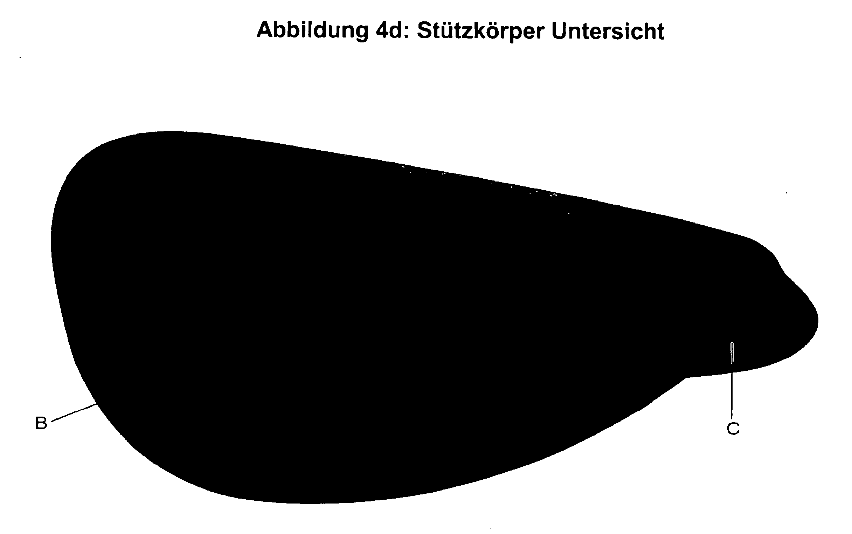

Die **Zone C** (Abb. 2, 3a, 4a, 4d) hat durch ihre halbeiförmig hervorgewölbte Gestalt die Funktion einen ausreichenden Anpressdruck, z. B. durch einen Slip zu gewährleisten und dient bei den Bauartvarianten auch als Flüssigkeitsabsorber.

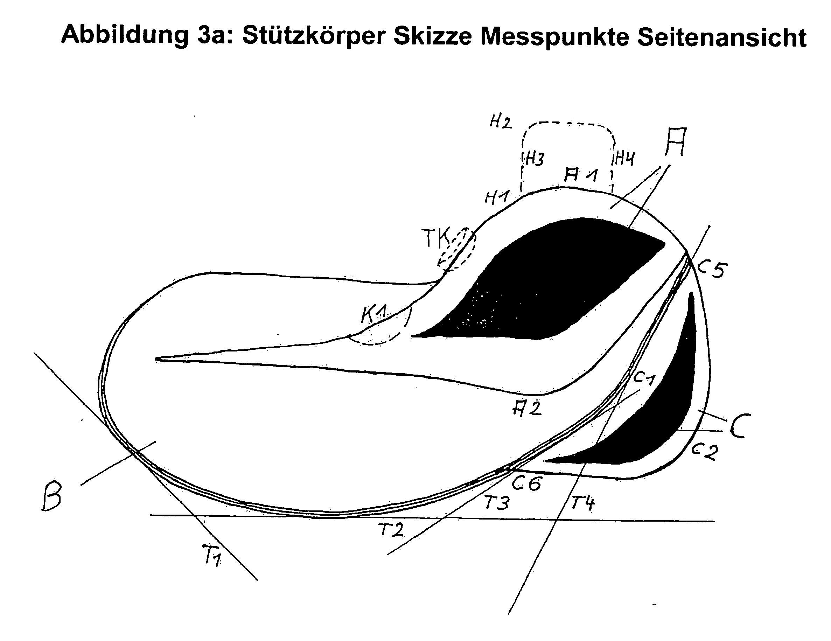

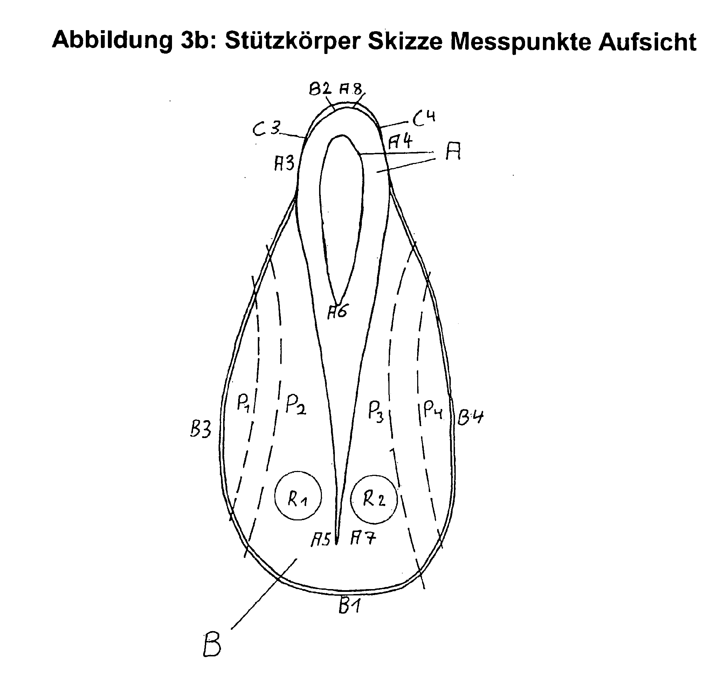

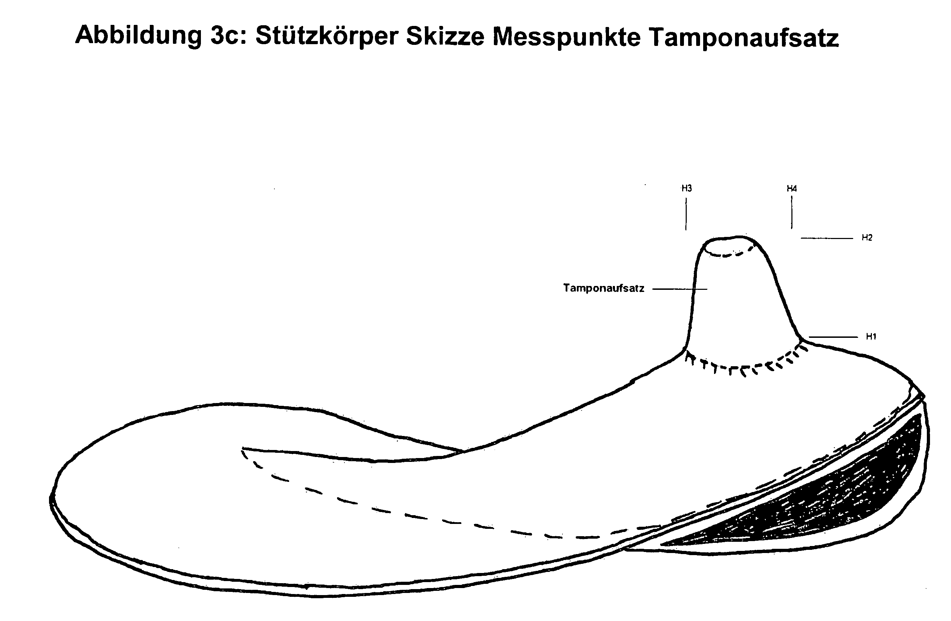

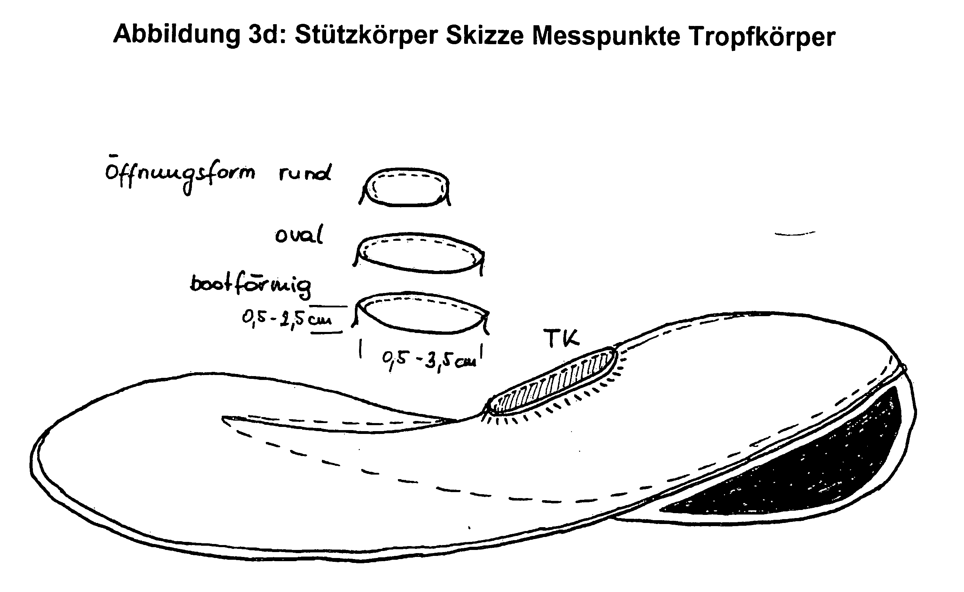

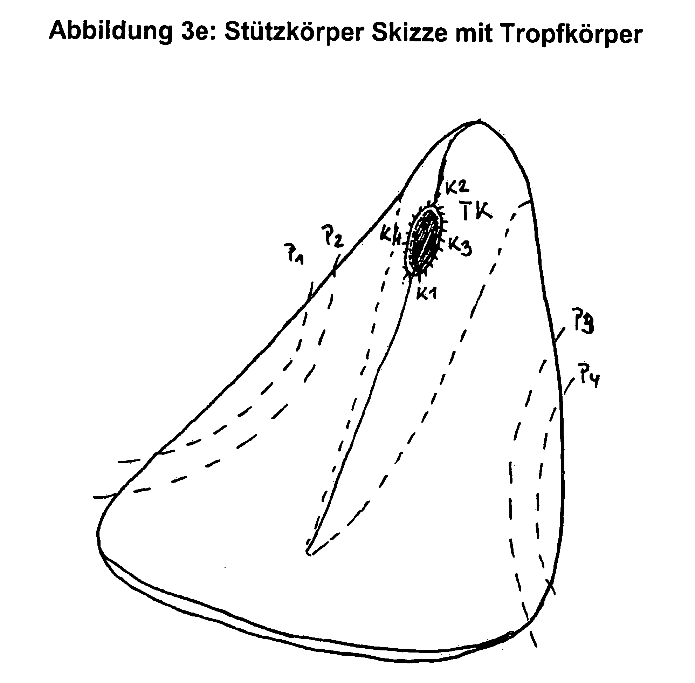

Die Außenseite des Stützkörpers besteht aus körperfreundlichem Kunststoff, dessen Oberfläche zur Erleichterung der Einführung in die Scheide "mikroskopisch glatt" ist, wobei das typische Rauhigkeitsprofil maximal +/- 5 µm betragen sollte. Dessen ungeachtet kann der Stützkörper gemäß Abb. 2, 3 a, 4a mit makroskopischen Profilen (z.B. Längs- und Quer-Riffelungen zur Erleichterung der Einführung oder zur besseren Positionierung) versehen werden, wobei solche Profile durch Höhenunterschiede im Bereich +/- 0.1 - 3 mm gekennzeichnet sind. Geeignet sind hier z. B. weiche Olefine wie z.B. LD-Polyethylen, sowie Latex- oder Silicongummi-Materialien. Optional können auf die Außenseite des Stützkörpers auch funktionelle Beschichtungen aufgebracht werden, die z.B. die Gleitfähigkeit verbessern oder bakterizid wirken.

Der Innenraum des Stützkörpers in Abb. 3a, 3b ist mit einem Material gefüllt, das bei minimalem Gewicht maximale Festigkeit gewährt. Aus Kostengründen bieten sich hier beispielsweise, aber nicht einschränkend, Schäume aus Polyurethan oder Polystyrol an.

Bei der Bauartvariante des Stützkörpers mit Tamponaufsatz gemäß Abb. 3c ist eine flüssigkeitsdurchlässige Gestaltung der scheidenseitigen Oberfläche des Stützkörpers erforderlich, die in bekannter Weise durchgeführt werden kann. Der Innenraum muss dann teilweise mit saugfähigen Materialien (z. B. auf der Basis von Zellulose oder den in Hygieneartikeln wie Windeln oder Damenbinden gebräuchlichen "Superabsorbern") ausgefüllt sein.

Bei der Bauartvariante des Stützkörpers als Urinabsorber gemäß Abb. 3d, 3e ist der Tropfkörper als flüssigkeitsaufnehmender Bereich, in runder, ovaler oder bootförmiger Ausführung, in der Oberfläche des Stützkörpers vorzusehen. Ein wulstförmiger Abschluss soll dabei eine abdichtende Funktion übernehmen. Auf eine Kapillarwirkung des flüssigkeitsübernehmenden Materials in Richtung Stützkörperinneres ist zu achten. Der Kern des Stützkörpers muss dann komplett mit saugfähigen Materialien, sogenannten Superabsorbern, ausgefüllt sein.

### Abbildungen:

Abb. 1a-c Übersichtsbild, Positionierung des erfindungsgemäßen Stützkörpers im Körper
Abb. 2 Stützkörperbereiche
Abb.3a Skizze des Stützbehelfs mit Messpunkte (Seitenansicht), Erläuterungen siehe Beschreibung und Patentansprüche
Abb. 3b Skizze des Stützbehelfs mit Messpunkte (Draufsicht), Erläuterungen siehe Beschreibung und Patentansprüche ,
Abb. 3c Skizze des Stützkörpers in Bauartvariante Tamponaufsatz (Seitenansicht), Erläuterungen siehe Beschreibung und Patentansprüche
Abb. 3d Skizze des Stützkörpers in Bauartvariante Tropfkörper (Seitenansicht), Erläuterungen siehe Beschreibung und Patentansprüche
Abb.4a Seitenansicht des Stützkörpers, Erläuterungen siehe Beschreibung und Patentansprüche
Abb. 4b Draufsicht von vorne auf den Stützkörper, Erläuterungen siehe Beschreibung und Patentansprüche
Abb. 4c Draufsicht von hinten auf den Stützkörper, Erläuterungen siehe Beschreibung und Patentansprüche
Abb. 4d Ansicht des Stützkörpers von unten, Erläuterungen siehe Beschreibung und Patentansprüche
Abb. 4e Seitliche Draufsicht des Stützkörpers, Erläuterungen siehe Beschreibung und Patentansprüche

## Patentansprüche

1. Monolithischer Stützkörper aus biegsamen Material zum Einführen in die vordere Scheide für die Repositionierung der Gebärmutter bei Uterus-prolaps, mit einer Formgebung die gedanklich in drei funktionelle Zonen A, B und C unterteilt werden kann, wobei die scheidenseitig gelegene Zone A die Form eines gebogenen langgestreckten Tropfens hat, die Zone B die Form eines gebogenen langgestreckten Herzens hat und die Zone C eine halbeiförmig gewölbte Gestalt hat, **dadurch gekennzeichnet, dass** die Zone A eine Einbuchtung (E) aufweist, die auf die Form der Klitoris abgestimmt ist, und dass auf der Zone A in Richtung Körper eine Ausstülpung (TK) in runder, ovaler oder bootförmiger Farm zur Aufnahme von Urin bei Inkontinenz ausgeformt ist, wobei die Ausstülpung eine Höhe (Strecke K3-K4) von 5-25 mm und eine Länge und Breite (Strecke K1-K2) von 5-35 mm aufweist.

2. Stützkörper nach Anspruch 1, **dadurch gekennzeichnet, dass** die scheidenseitig gelegene Zone A eine Höhe (Strecke A₁-A₂) von 15-65 mm hat, am hinteren (anusseitigen) Ende eine Breite (Strecke A₃-A₄) von 15-30 mm hat, am vorderen Ende eine Breite (Strecke A₅-A₆) von 5-30 mm hat und eine Länge (Strecke A₇-A₈) von 20-130 mm hat.

3. Stützkörper nach Anspruch 1 oder 2, **dadurch gekennzeichnet, dass** die Zone B in der Projektion eine Länge (Strecke B₁-B₂) von 20-150 mm hat, eine maximale Breite (Strecke B₃-B₄) von 30-100 mm hat und dass der Schnittpunkt der Länge (Strecke B₁-B₂) und der Breite (Strecke B₃-B₄) die Länge (Strecke B₁-B₂) im Verhältnis von 20:80 bis 30:70 teilt.

4. Stützkörper nach einem der Ansprüche 1 bis 3, **dadurch gekennzeichnet dass** die Tangenten T₁ und T₂ gemäß Abb. 3a, 4a einen Winkel α von 20-55 Grad, die Tangenten T₂ und T₃ gemäß Abb. 3a, 4a einen Winkel β von 10-35 Grad und die Tangenten T₂ und T₄ gemäß Abb. 3a, 4a einen Winkel γ von 50-60 Grad einschließen.

5. Stützkörper nach einem der Ansprüche 1 bis 4, **dadurch gekennzeichnet, dass** die langgestreckte Tropfenform der Zone B durch Einbuchtungen (gestrichelte Linien P₁-P₂ und P₃-P₄) modifiziert wird.

6. Stützkörper nach einem der Ansprüche 1 bis 5, **dadurch gekennzeichnet, dass** körperseitig haftungsverbessernde Rauh- oder Adhesionsbereiche aufgebracht sind. (Bereiche R₁ und R₂).

7. Stützkörper nach einem der Ansprüche 1 bis 6, **dadurch gekennzeichnet, dass** die außenseitig gelegene halbeiförmig gewölbte Zone C eine Höhe (Strecke C₁-C₂) von 10-55 mm hat, eine Breite (Strecke C₃-C₄) von 5-30 mm hat und eine Länge (Strecke C₅-C₆) von 20-150 mm hat.

8. Stützkörper nach einem der Ansprüche 1 bis 7, **dadurch gekennzeichnet, dass** die drei Zonen A, B und C eine Einheit ohne Trennflächen bilden und aus dem gleichen Material bestehen.

9. Stützkörper nach einem der Ansprüche 1 bis 8, **dadurch gekennzeichnet dass** der Innenraum des Stützkörpers aus geschäumten Kunststoff besteht.

10. Stützkörper nach einem der Ansprüche 1 bis 9, **dadurch gekennzeichnet, dass** die Zonen A, B und C nicht aus dem gleichen Material bestehen, jedoch fest miteinander verbunden sind.

11. Stützkörper nach einem der Ansprüche 1 bis 10, **dadurch gekennzeichnet, dass** die scheidenseitige Oberfläche durchlässig für Körperflüssigkeiten ausgelegt wird.

12. Stützkörper nach einem der Ansprüche 1 bis 11, **dadurch gekennzeichnet, dass** sein Innenraum mit hygroskopisch saugfähigen Materialien ausgefüllt ist, die in der Lage sind, Körperflüssigkeiten aufzunehmen.

13. Stützkörper nach einem der Ansprüche 1 bis 12, **dadurch gekennzeichnet, dass** die Zone A in Richtung des Körperinneren in Form eines, an der Spitze abgerundeten, mit saugfähigen Materialien gefüllten zylindrischer Hohlkörpers ausgeformt ist, wobei der zylinderische Hohlkörper im Wesentlichen die Form eines konventionellen Tampons mit einer Höhe (Strecke H₁-H₂) von 5-50 mm und einem Durchmesser (Strecke H₃-H₄) von 5-20 mm aufweist.

## Claims

1. A monolithic support body made of flexible material for insertion into the front vagina for the repositioning of the uterus in the case of a prolapse of the uterus, having a shaping which can be subdivided theoretically into three functional zones A, B and C, with the zone A on the side of the vagina having the shape of a curved elongated drop, zone B the shape of a curved elongated heart and zone C the shape of a half-egg-shaped curved form, **characterized in that** zone A comprises an indentation (E) which is adjusted to the shape of the clitoris, and on zone A in the direction of the body there is formed a protuberance (TK) in round, oval or boot-like shape for receiving urine in the case of incontinence, with the protuberance having a height (line K3-K4) of 5 to 25 mm and a length and width (line K1-K2) of 5 to 35 mm.

2. A support body according to claim 1, **characterized in that** the zone A on the side of the vagina has a height (line A₁-A₂) of 15 to 65 mm, a width (line A₃-A₄) of 15 to 30 mm at the rear end (on the anus side), a width (line A₅-A₆) of 5 to 30 mm at the front end, and a length (line A₇-A₈) of 20 to 130 mm.

3. A support body according to claim 1 or 2, **characterized in that** the zone B has a length (line B₁-B₂) of 20 to 150 mm in projection, a maximum width (line B₃-B₄) of 30 to 100 mm, and the point of intersection of the length (line B₁-B₂) and width (line B₃-B₄) divides the length (line B₁-B₂) at a ratio of 20:80 to 30:70.

4. A support body according to one of the claims 1 to 3, **characterized in that** the tangents T₁ and T₂ according to Figs. 3a, 3b enclose an angle α of 20 to 55 degrees, the tangents T₂ and T₃ according to Figs. 3a, 3b enclose an angle β of 10 to 35 degrees, and the tangents T₂ and T₄ according to Figs. 3 a, 3b enclose an angle γ of 50 to 60 degrees.

5. A support body according to one of the claims 1 to 4, **characterized in that** the elongated shape of a drop of zone B is modified by indentations (broken lines P₁-P₂ and P₃-P₄).

6. A support body according to one of the claims 1 to 5 **characterized in that** rough or adhesion regions that improve adhesion on the body side are applied (regions R₁ and R₂).

7. A support body according to one of the claims 1 to 6, **characterized in that** the half-egg shaped zone C disposed on the outside has a height (line C₁-C₂) of 10 to 55 mm, a width (line C₃-C₄) of 5 to 30 mm and a length (line C₅-C₆) of 20 to 150 mm.

8. A support body according to one of the claims 1 to 7, **characterized in that** the three zones A, B and C form a unit without any separating surfaces and consist of the same material.

9. A support body according to one of the claims 1 to 8, **characterized in that** the interior space of the support body consists of Foamed plastic.

10. A support body according to one of the claims 1 to 9, **characterized in that** the zones A, B and C do not consist of the same material, but are rigidly connected with each other.

11. A support body according to one of the claims 1 to 10, **characterized in that** the surface on the vagina side is configured to be permeable for bodily fluids.

12. A support body according to one of the claims 1 to 11, **characterized in that** its interior space is filled with hygroscopic absorbent materials which are capable of absorbing bodily fluids.

13. A support body according to one of the claims 1 to 12, **characterized in that** the zone A is formed in the direction of the interior of the body in the shape of a cylindrical hollow body which is filled with absorbent materials and is rounded off at the tip, with the cylindrical hollow body substantially having the shape of a conventional tampon with a height (line H₁-H₂) of 5 to 50 mm and a diameter (line H₃-H₄) of 5 to 20 mm.

## Revendications

1. Élément de soutien monolithique en matière flexible destiné à être inséré dans la partie antérieure du vagin pour le repositionnement de l'utérus en cas de prolapsus utérin, avec une conformation qui peut être partagée fictivement en trois zones fonctionnelles A, B et C, la zone A située du côté du vagin ayant la forme d'une goutte allongée et incurvée, la zone B la forme d'un coeur allongé et incurvé et la zone C une forme bombée semi-ovoïde, **caractérisé en ce que** la zone A présente un renfoncement (E) adapté à la forme du clitoris et **en ce qu'**il est prévu formé sur la zone A en direction du corps une saillie (TK) de forme ovale ou naviculaire destinée à recueillir l'urine en cas d'incontinence, cette saillie ayant une hauteur (distance K₃-K₄) de 5 à 25 mm et une longueur et une largeur (distance K₁-K₂) de 5 à 35 mm.

2. Élément de soutien selon la revendication 1, **caractérisé en ce que** la zone A située du côté du vagin a une hauteur (distance A₁-A₂) de 15 à 65 mm, une largeur à l'extrémité arrière (du côté de l'anus, distance A₃-A₄) de 15 à 30 mm, une largeur à l'extrémité antérieure (distance A₅-A₆) de 5 à 30 mm et une longueur (distance A₇-A₈) de 20 à 130 mm.

3. Élément de soutien selon la revendication 1 ou 2, **caractérisé en ce que** la zone B a en projection une longueur (distance B₁-B₂) de 20 à 150 mm, une largeur maximale (distance B₃-B₄) de 30 à 100 mm, et **en ce que** l'intersection entre la longueur (distance B₁-B₂) et la largeur (distance B₃-B₄) partage la longueur (distance B₁-B₂) dans une proportion de 20/80 à 30/70.

4. Élément de soutien selon l'une des revendications 1 à 3, **caractérisé en ce que** les tangentes T₁ et T₂ selon les Figures 3a, 4a forment un angle α de 20° à 55°, les tangentes T₂ et T₃ selon les Figures 3a, 4a un angle β de 10° à 35° et les tangentes T₂ et T₄ selon les Figures 3a, 4a un angle γ de 50° à 60°.

5. Élément de soutien selon l'une des revendications 1 à 4, **caractérisé en ce que** la forme de goutte allongée de la zone B est modifiée par des renfoncements (lignes de pointillés P₁-P₂ et P₃-P₄).

6. Élément de soutien selon l'une des revendications 1 à 5, **caractérisé en ce que** des zones rugueuses ou adhérentes améliorant l'adhérence sont appliquées du côté du corps (zones R₁ et R₂).

7. Élément de soutien selon l'une des revendications 1 à 6, **caractérisé en ce que** la zone C bombée de forme semi-ovoïde située du côté de l'extérieur a une hauteur (distance C₁-C₂) de 10 à 55 mm, une largeur (distance C₃-C₄) de 5 à 30 mm et une longueur (distance C₅-C₆) de 20 à 150 mm.

8. Élément de soutien selon l'une des revendications 1 à 7, **caractérisé en ce que** les zones A, B et C forment une unité sans surfaces de séparation et sont faites du même matériau.

9. Élément de soutien selon l'une des revendications 1 à 8, **caractérisé en ce que** l'intérieur de l'élément de soutien se compose de mousse de matière plastique.

10. Élément de soutien selon l'une des revendications 1 à 9, **caractérisé en ce que** les zones A, B et C ne sont pas faites du même matériau mais sont reliées entre elles de manière fixe.

11. Élément de soutien selon l'une des revendications 1 à 10, **caractérisé en ce que** la surface du côté du vagin est perméable aux fluides corporels.

12. Élément de soutien selon l'une des revendications 1 à 11, **caractérisé en ce que** son intérieur est rempli de matériau absorbant hygroscopique, qui est capable d'absorber les fluides corporels.

13. Élément de soutien selon l'une des revendications 1 à 12, **caractérisé en ce que** la zone A est conformée, en direction de l'intérieur du corps, comme un corps creux cylindrique arrondi à son extrémité et rempli de matériau absorbant, lequel corps creux cylindrique a sensiblement la forme d'un tampon hygiénique conventionnel avec une hauteur (distance H₁-H₂) de 5 à 50 mm et un diamètre (distance H₃-H₄) de 5 à 20 mm.
